(19) Europäisches Patentamt
European Patent Office
Office européen des brevets

(11) Publication number: **0 338 215 A2**

(12) **EUROPEAN PATENT APPLICATION**

(21) Application number: 89103250.0

(51) Int. Cl.4: C07C 63/68 , C07C 51/567

(22) Date of filing: 24.02.89

The title of the invention has been amended (Guidelines for Examination in the EPO, A-III, 7.3).

(30) Priority: 24.02.88 US 160034

(43) Date of publication of application: 25.10.89 Bulletin 89/43

(84) Designated Contracting States: AT BE CH DE ES FR GB IT LI NL SE

(71) Applicant: OCCIDENTAL CHEMICAL CORPORATION
P.O. Box 189
Niagara Falls New York 14302(US)

(72) Inventor: Spohn, Ronald F.
60 Charter Oaks
Williamsville New York(US)

(74) Representative: Vossius & Partner
Siebertstrasse 4 P.O. Box 86 07 67
D-8000 München 86(DE)

(54) **Process for the preparation of halogen-substituted phthalic anhydride.**

(57) A process for the preparation of halogen substituted phthalic anhydrides of the formula:

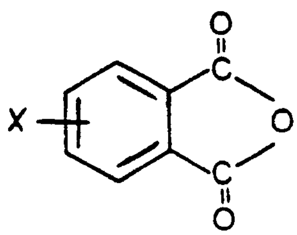

wherein X is a halogen, by the reaction of a halogen substituted tetrahydrophthalic anhydride of the formula:

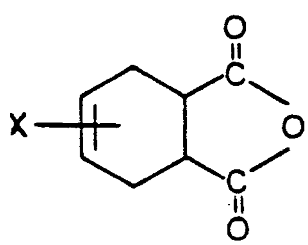

or a geminal dihalogen substituted hexahydrophthalic anhydride of the formula:

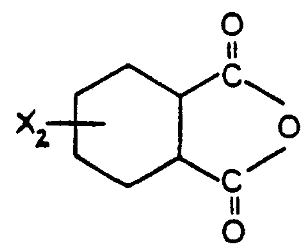

in a vapor or melt phase while mixed with a halogen at a temperature of 200-500° C.

EP 0 338 215 A2

# SELECTIVE DEHYDROGENATION WITH HALOGENS

This invention is concerned with the process for the aromatization of a cyclic compound to form a fully aromatic ring structure. More particularly, it relates to a process for producing a halogen substituted phthalic anhydride from a halogen substituted tetrahydrophthalic anhydride or from a dihalogen substituted hexahydrophthalic anhydride.

Substituted phthalic anhydrides are valuable raw materials for the synthesis of useful products. These anhydrides are utilized as intermediates in the synthesis of organic polymers, dyes, plasticizers and in other uses.

Procedures are known for preparing substituted phthalic anhydrides. U.S. Patent 4,560,772 discloses the reaction of 4-methyltetrahydrophthalic anhydride with excess sulfur and a catalytic amount of zinc oxide and 2-mercaptobenzothiazole to produce 4-methylphthalic anhydride and hydrogen sulfide.

U.S. Patent 4,560,773 discloses a similar reaction between the electron rich 4-methyltetrahydrophthalic anhydride and bromine in the presence of a catalytic amount of an acid acceptor such as dimethylformamide or pyridine in the liquid phase.

U.S. Patent No. 4,709,056 discloses the dehydrohalogenation of dihalohexahydrophthalic anhydrides through the use of a basic alumina catalyst in a liquid phase to produce 4-fluoro-1,2,3,6-tetrahydrophthalic anhydride.

Ohkatou et al, J. Japan Petrol. Inst., 22, 164-9, (1979), discloses the dehydrogenation of hydrocarbons using an activated carbon bed to produce the corresponding olefins. The mechanism of the reaction using cyclohexane and cyclohexene were studied using a pressure flow technique.

Bergmann, J. Amer. Chem. Soc., 64, 176, (1942), discloses the aromatization of tetrahydrophthalic anhydride products of Diels-Alder reactions. The author discloses that dehydrogenation occurs when the tetrahydrophthalic anhydride product is boiled in nitrobenzene. However, it is further disclosed that dehydrogenation does not occur when p-bromonitrobenzene, p-chloronitrobenzene, or m-dinitrobenzene in xylene is employed. Moreover, it has been found that when the dihalohexahydrophthalic anhydrides of this publication are dehydrogenated in nitrobenzene, a portion of the nitrobenzene is reduced to aniline. The aniline reacts with the anhydride group of either the starting material or product to form imides and thus lower the yield of desired product.

Skvarchenko et al, Obshchei Khimiil, Vol. 30, No. 11, pp 3535-3541 (1960), disclose the aromatization of chlorosubstituted tetrahydrophthalic anhydride by heating with phosphorus pentoxide. In the aromatization process described, however, decarboxylation also occurs with the formation of the corresponding chlorosubstituted benzene compound. The preparation of tetrahydrophthalic acids and anhydrides and various methods for dehydrogenation and aromatization thereof are reviewed by Skvarchenko in Russian Chemical Reviews, Nov. 1963, pp 571-589.

The aromatization of organic compounds has been shown by various techniques and the use of special catalysts seem to control the satisfactory conversion at useful product yields and conversion times. This study was undertaken to determine if a vapor state aromatization of halogen substituted materials such as halogenated tetrahydrophthalic anhydrides or geminal dihalogenated hexahydrophthalic anhydrides could convert the material to a monohalogenated phthalic anhydride at adequate yields and within a reasonable time.

It is the principal object of the present invention to provide novel intermediate compounds which are useful and provide a commercially attractive synthetic route for the preparation of a halogen substituted phthalic anhydride from a halogen substituted tetrahydrophthalic anhydride, or from a geminal dihalogen substituted hexahydrophthalic anhydride.

The reaction can be conducted either in the vapor phase or in a liquid (melted) state by the passage of a halogen through the reaction zone at an elevated temperature. The temperature is in the range of 200 to 500° C.

A specific example of the reaction employs 4-chlorotetrahydrophthalic anhydride as a starting material and yields 4-chlorophthalic anhydride as the end product in high yield.

This invention is concerned with the aromatization of a cyclic compound to form a fully aromatized ring and specifically to convert a halogen substituted tetrahydrophthalic anhydride or a dihalogenated hexahydrophthalic anhydride into a halogen substituted phthalic anhydride. The aromatization reaction occurs in either the vapor phase by the passage through the reaction zone using a halogen flow and an inert gas, e.g., nitrogen to assist in the passage of the reactant through the heated zone or in the liquid or melt phase.

The starting material employed in this invention can be prepared by various known methods, and many such compounds are commercially available. Diels-Alder addition reaction of a maleic anhydride with a conjugated diene is a typical reaction which produces such an anhydride with a partially saturated six membered ring. Depending upon the desired product, the diene and/or the maleic anhydride can contain substituents to provide the starting material for this invention. The following halogen substituted partially saturated phthalic anhydrides can be employed but the invention is not limited to the following:

4-chloro-1,2,3,6-tetrahydrophthalic anhydride;
4-fluoro-1,2,3,6-tetrahydrophthalic anhydride;
4-bromo-1,2,3,6-tetrahydrophthalic anhydride;
4-chloro-1,2,5,6-tetrahydrophthalic anhydride;
4-fluoro-1,2,5,6-tetrahydrophthalic anhydride;
4-bromo-1,2,5,6-tetrahydrophthalic anhydride;
4,4-difluorohexahydrophthalic anhydride;
4,4-dichlorohexahydrophthalic anhydride;
4,4-dibromohexahydrophthalic anhydride;
3-chloro-1,2,5,6-tetrahydrophthalic anhydride;
3-fluoro-1,2,5,6-tetrahydrophthalic anhydride;
3-bromo-1,2,5,6-tetrahydrophthalica anhydride;
3,3-difluorohexahydrophthalic anhydride;
3,3-dichlorohexahydrophthalic anhydride;
3,3-dibromohexahydrophthalic anhydride; the corresponding iodide derivatives can also be included.

The aromatization reaction can be conducted in the vapor phase. This reaction is conducted using a metallic tube, preferably of a nickel base and is heated to a temperature to assist in the vaporization of the components. The procedure involved heating, for example, 4-chloro-1,2,3,6,-tetrahydrophthalic anhydride until it melts and then with the aid of a pump, is pumped into the reaction tube, where it is vaporized. A halogen is also passed into the heated reaction tube. The two gases mix and react as they passed through the heated tube. The products were vented from the tube into a cooler container which allowed the collection of the 4-chlorophthalic anhydride. The halogen acid produced as a side product, is trapped in an aqueous media.

Placed into the metallic reactor tube may be a portion of nickel mesh, which serves to increase the surface area and provide additional mixing for the gases.

If the liquid or melt phase system is used, the procedure involves the melting of the starting material, for example, of 4-chloro-1,2,3,6-tetrahydrophthalic anhydride in a reactor containing a gas inlet tube, a stirrer, condenser, and an outlet connected to a trap. The reactor is heated to melt the material and a continuous flow of halogen, e.g., chlorine is passed through the stirred melt and the product collects in the trap. The reactor may or may not be illuminated with an ultraviolet lamp (300-400nm) during the reaction.

In the above description, the halogen used as the hydrogen acceptor was identical to the halogen on the substituted tetrahydrophthalic anhydride starting material, but this need not be the case. Any halogen excluding fluorine will function as a hydrogen acceptor. If a different halogen is used, small amounts of mixed halogen side products can be produced. The major product will be the phthalic anhydride with the same halogen as the starting material. Depending upon the starting material, e.g., a monohalotetrahydrophthalic anhydride, will require two moles of halogen gas for each mole of starting material. The dihalogen substituted hexahydrophthalic anhydride will require three moles for each mole of starting material. However, a slight excess of halogen gas is used to facilitate the reaction in the vapor or liquid phase.

A solvent can be used in the reaction, but is generally not preferred. When a solvent is used, the starting material is dissolved in the solvent prior to introduction of the starting material to the reactor. In the vapor phase reaction, a lower boiling solvent is preferred, such as toluene or ethyl acetate. In the melt or liquid phase reaction, a higher boiling solvent such as 1,2,4-trichlorobenzene is preferred.

## Example I

A U-shaped nickel reactor tube, 3/4 inch ID, 6 inches long on each side, was wrapped with heating tapes. Placed within the outlet leg of the tube were nickel mesh to increase the surface area, and thermocouples to measure the temperature. At the feed end of the reaction tube are two openings, one for the halogen feed and the second, for the raw material feed. To the outlet of the reactor is placed a series of receivers and traps to collect the product and entrap the acid side product. A quantity of 4-chlorotetrahydrophthalic anhydride was placed in a flask and heated, the melted material was transferred, in a steady flow by means of a pump, through heated lines to the inlet of the reactor tube. A flow of dry nitrogen was used to assist in the passage of materials through the reactor (20 ml/mm). The raw material was vaporized at 225 °C. The reactor temperature was 240 to 300 °C. Chlorine flow was 230 ml/min, and the organic flow was 0.15 grams/minute. The ratio of the flow of chlorine and the flow of organic were set at 2.5 moles of chlorine per mole of organic. The reaction was conducted under these temperatures and conditions and a product of 4-chlorophthalic anhydride was obtained at a 50% yield.

## Example II

To a three necked 50 ml round bottom flask, fitted with a stirrer, gas inlet tube and connected to a trap system, was placed 20.38 g of 4-chloro-1,2,3,6-tetrahydrophthalic anhydride. The reactor

was heated to 200°C and illuminated with a 15 watt black, blue bulb (GE F15T8-BLB) with a transmission range of 300-400 nm and a maximum at 375 nm. An excess flow of chlorine was begun and the reaction continued for 8 hours. The reactor was purged with nitrogen and sampled. A 12% yield of 4-chlorophthalic anhydride was obtained.

While the invention has been described in connection with specific embodiments thereof, it will be understood that it is capable of further modification, and this application is intended to cover any variation, uses, or adaptations of the invention following, in general, the principles of the invention and including such departures from the present disclosure has come within known or customary practice in the art to which the invention pertains and as may be applied to the essential features hereinbefore set forth, as fall within the scope of the invention.

## Claims

1. A process for the preparation of halogen substituted phthalic anhydride of the formula:

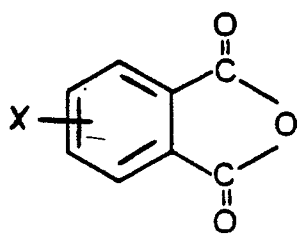

where X is a halogen, by reacting either a monohalogen substituted tetrahydrophthalic anhydride of the formula:

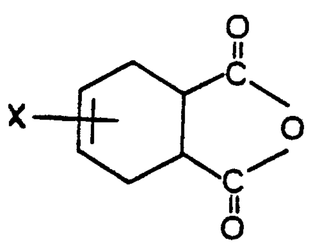

or a geminal dihalogen substituted hexahydrophthalic anhydride of the formula:

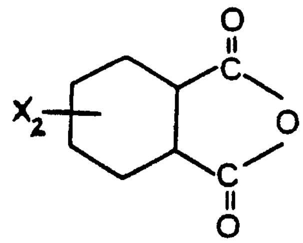

admixed with a halogen gas

a) in the vapor phase at a temperature of 200-500°C; or

b) in a liquid phase at a temperature of 200-300°C.

2. The process of Claim 1, wherein the tetrahydrophthalic anhydride is 4-chlorotetrahydrophthalic anhydride.

3. The process of Claim 1, wherein the tetrahydrophthalic anhydride is 3-chlorotetrahydrophthalic anhydride.

4. A process of Claim 1, wherein the phthalic anhydride product is 4-chlorophthalic anhydride.

5. A process of Claim 1, wherein the phthalic anhydride product is 3-chlorophthalic anhydride.

6. A process of Claim 1, wherein the tetrahydrophthalic anhydride is 4-fluorotetrahydrophthalic anhydride.

7. A process of Claim 1, wherein the phthalic anhydride product is 4-fluorophthalic anhydride.

8. A process of Claim 1, wherein the tetrahydrophthalic anhydride is 4-bromotetrahydrophthalic anhydride.

9. A process of Claim 1, wherein the phthalic anhydride product is 4-bromophthalic anhydride.

10. A process of Claim 1, wherein the hexahydrophthalic anhydride is 4,4-difluorohexahydrophthalic anhydride.

11. A process of Claim 1, wherein the reaction is conducted in the vapor phase in the range of 200-325°C.

12. A process of Claim 1, wherein the reaction is conducted in a liquid phase in the range of 200-250°C.